# EUROPÄISCHE PATENTSCHRIFT

(11) **EP 4 026 507 B1**
(45) Veröffentlichungstag und Bekanntmachung des Hinweises auf die Patenterteilung: **09.12.2026**
(21) Anmeldenummer: 21151169.6
(22) Anmeldetag: 12.01.2021
(51) Int. Cl.: A61B 18/14

(54) **DISSEKTIONSINSTRUMENT**
DISSECTION INSTRUMENT
INSTRUMENT DE DISSECTION

(43) Veröffentlichungstag der Anmeldung: 13.07.2022
(73) Patentinhaber: Erbe Elektromedizin GmbH, 72072 Tübingen (DE)
(72) Erfinder: Löser, David, 72108 Rottenburg am Neckar (DE)
(74) Vertreter: Rüger Abel IP Legal Solutions GmbH

(56) Entgegenhaltungen:
- EP-A1- 3 132 765
- EP-A1- 3 138 522
- JP-A- 2004 180 843

## Beschreibung

Die Erfindung betrifft ein elektrochirurgisches Instrument, das zur Dissektion oder zur Gewebefusion oder Gewebekoagulation insbesondere kombiniert mit der Gewebedissektion eingerichtet und geeignet ist.

Die JP 2004180843 A offenbart ein Gewebekoagulations- und Dissektionsinstrument mit zwei Branchen nach Art einer Zange mit gegeneinander beweglichen Branchen, zwischen denen Gewebe gefasst werden kann. Die beiden Branchen sind bestrombar, um zwischen einander gefasstes Gewebe zu koagulieren. Außerdem weist die eine der Branchen eine Schneidelement auf, dem ein elastisches an der anderen Branche angeordnetes Widerlager zugeordnet ist. Zum Widerlager werden verschiedene Bauformen vorgeschlagen. Beispielsweise kann das elastische Widerlager einen quadratischen Vollquerschnitt, einen Hohlquerschnitt oder einen abgestuften Querschnitt nach Art eines flachen T aufweisen und in eine unten offene Nut der unteren Branche eingelegt sein. Dieses Widerlager weist einen oberen der dem Schneidelement zugewandten breiteren Abschnitt und einen schmaleren sich in die Nut hinein erstreckenden Abschnitt auf. Beim Schließen der Branche verformt sich das Widerlager in die Nut hinein. Während der mittlere Bereich des Widerlagers nirgendwo anliegt, sind die Ränder des Widerlagers an Stufen gefasst und abgestützt, die sich entlang der mittleren Nut erstrecken.

Aus der US 2004/0049185 A1 ist ein Koagulations- und Dissektionsinstrument mit elastischem Widerlager bekannt. Das Widerlager weist einen etwa quadratischen Querschnitt auf und stützt sich an seiner der Schneidelektrode gegenüber liegenden Seite an der ansonsten U-förmig ausgebildeten Branche zwischen ihren beiden Schenkeln ab. Die Flanken des Widerlagers liegen frei.

Die US 2009/0234355 A1 offenbart ein Koagulations- und Dissektionsinstrument mit einer in einer Branche angeordneten Schneidelektrode und einem in der anderen Branche angeordneten beweglich gelagerten Widerlager. Diese ist federnd unterstützt, um der eindringenden Schneidelektrode ausweichen zu können.

Weitere Beispiele für elektrochirurgische Instrumente mit Koagulationselektroden, Schneidelektroden und den Schneidelektroden zugeordneten Widerlagern können der EP 1 632 192 A1 und der EP 2 754 403 A2 entnommen werden.

Die Anforderungen an Koagulations- und Dissektionsinstrumente steigen in mehrerlei Hinsicht. Es gibt Miniaturisierungstendenzen sowie Anforderungen an Fertigungssicherheit und Fertigungspräzision.

Davon ausgehend, ist es Aufgabe der Erfindung, ein Instrument zu schaffen, das in mindestens einer Hinsicht verbessert ist.

Diese Aufgabe erfüllt das erfindungsgemäße Instrument nach Anspruch 1:

Das erfindungsgemäße Instrument weist an einer Branche eine Schneidelektrode und an der anderen gegenüberliegenden Branche ein elastisches Widerlager auf. Das Widerlager ist in einem Rahmen gehalten, der von der betreffenden Branche selbst oder einem gesonderten Bauteil gebildet ist. Das Widerlager weist eine Umfangsfläche auf, die an den Rahmen anschließt und dort stoffschlüssig durch Anspritzen mit dem Rahmen zugfest verbunden ist. Der Rahmen fixiert das Widerlager wenigstens an zwei einander gegenüberliegenden Seiten. Vorzugsweise umschließt der Rahmen das Widerlager dreiseitig, d.h. an seinen beiden Flanken und an dem distalen Ende. Das Widerlager ist zumindest an seinen beiden langen Seiten oder Flanken und wahlweise auch an seinem distalen Ende mit dem Rahmen verbunden. In letzterem Fall verschließt das Widerlager den Rahmen komplett und lässt kein Material in den Bereich unterhalb des Widerlagers gelangen. Es ist aber auch möglich, die Membran an ihrem distalen Ende und/oder an ihrem proximalen Ende frei, d.h. nicht mit dem Rahmen verbunden, zu lassen.

Dem Widerlager zugewandt weist der Rahmen eine Fläche auf, die mit der Umfangsfläche des Widerlagers verbunden ist. Die Verbindung ist zugfest, d.h. eine von dem Rand des Widerlagers zu seiner Mitte hin gerichtete Zugkraft löst das Widerlager nicht von dem Rahmen. Die Verbindung ist vorzugsweise spaltfrei, d.h. die Umfangsfläche des Widerlagers ist vollflächig mit dem Rahmen verbunden. Unter Zugbelastung hebt vorzugsweise kein Teil des Widerlagers von dem Rahmen ab. Eine Zugkraft kann entstehen, indem die Schneidelektrode sowie gegebenenfalls zwischen Schneidelektrode und Widerlager vorhandenes biologisches Gewebe beim Schließen der Branche auf das Widerlager drückt. Dieses wird dabei aus seiner Ruheposition ausgelenkt und gespannt. Dabei ist das Widerlager vorzugsweise so flexibel ausgebildet, dass durch die Deformation des Widerlagers hauptsächlich Zugspannungen entstehen, weswegen das Widerlager auch als "Membran" bezeichnet wird. Im bevorzugten Fall ist das Widerlager so gestaltet, dass es sich hinsichtlich seiner mechanischen Spannungen im nicht belasteten Zustand bei offenen Branchen und vorzugsweise auch im belasteten Zustand bei geschlossenen Branchen im sogenannten Membranzustand befindet, d.h. die im Material des Widerlagers vorhandenen Zugspannungen sind zumindest näherungsweise überall gleich.

Die zugfeste Verbindung zwischen den Widerlager und dem Rahmen ist vorzugsweise eine stoffschlüssige Verbindung, gegebenenfalls eine rein stoffschlüssige Verbindung. Sie kann auf einfachste Weise zustande kommen, indem die Membran im Spritzgussverfahren erzeugt wird, wobei der Rahmen einen Teil der Spritzgussform bildet, d.h. den Materialfluss des noch flüssigen Widerlagers an der Umfangfläche des entstehenden Widerlagers begrenzt. Der Rahmen besteht vorzugsweise aus Metall. Das Widerlager besteht vorzugsweise aus flexiblem Kunststoff beispielsweise einem Silikonkunstoff. Die Haftung zwischen der Umfangsfläche der Membran und dem Rahmen kann durch entsprechende Oberflächengestaltung der der Membran zugewandten Fläche des Rahmens, zum Beispiel durch eine Aufrauhung, erhöht werden. Außerdem kann die Oberfläche des Rahmens zur Verbesserung der Haftung des Widerlagers vollständig oder abschnittsweise aktiviert werden. Dazu kann die Oberfläche stellenweise oder ganz einem CVD- oder PVD-Beschichtungsverfahren unterzogen werden. Ist der Rahmen ein Stanzteil, können beim Stanzen entstehenden Oberflächenunebenheiten des Rahmens zur Verbesserung der Haftung zwischen dem Rahmen und der Membran genutzt werden.

Die zugfeste Verbindung zwischen dem Widerlager und dem Rahmen kann durch Formschluss zwischen Widerlager und Rahmen unterstützt werden, beispielsweise indem der Rahmen entlang seiner dem Widerlager zugewandten Fläche Formschlussstrukturen aufweist, wie beispielsweise Öffnungen, Ausschnitte oder dergleichen.

Vorzugsweise ist das Widerlager in dem Rahmen unterstützungsfrei gehalten. Insbesondere liegt es an keiner der Eindringrichtung der Schneidelektrode entgegen gerichteten Fläche auf. Mit anderen Worten, die der Schneidelektrode zugewandte freie Oberseite ist ebenso groß wie die von der Schneidelektrode abgewandt angeordnete freie Unterseite des Widerlagers. Infolge dieser Anordnung wird zwischen den Branchen gefasstes biologisches Gewebe besonders sanft und gleichmäßig an die Schneidelektrode gedrückt, sodass das zwischen der Schneidelektrode und dem Widerlager gefasste Gewebe elektrisch geschnitten, nicht aber mechanisch gequetscht wird. Der Trennvorgang ist ein rein elektrischer. Dies lässt sich sowohl bei großen Bauformen des Instruments als auch bei miniaturisierten Bauformen desselben erreichen.

Das Widerlager besteht vorzugsweise aus einem elektrisch isolierenden Kunststoff, wie z.B**.** Silicon. Es kann aber auch aus einem Kunststoff bestehen, der intrinsisch oder extrinsisch, d.h. durch Einlagerung elektrisch leitfähiger Stoffe oder Partikel elektrisch leitfähig ist. Dadurch kann während des Stromdurchflusses durch das Gewebe eine zusätzliche thermische Wirkung entstehen.

Vorzugsweise weist das Widerlager zwei Flanken auf, die in konstantem Abstand zueinander angeordnet sind. Der Abstand kann aber auch in distaler Richtung abnehmen.

Das Widerlager kann in seinem mittleren Bereich, der im geschlossenen Zustand die Schneidelektrode berührt, eine Dicke aufweisen, die höchsten so groß ist, wie die Dicke am Rand des Widerlagers. Vorzugsweise ist die Dicke im mittleren Bereich sogar geringer als am Rand des Widerlagers. Dadurch wird ein Hauptvorteil der Erfindung gefördert. Beim Schließen des Instruments wird das Widerlager vorzugsweise auf Dehnung beanstandet und weniger auf Stauchung oder Biegung wie es beim Stand der Technik häufig der Fall war. Damit kann sich das membranartige Widerlager auch an inhomogenes Gewebe gut anpassen und dieses in sanfter Anlage an der Schneidelektrode halten. Es ist aber auch möglich, die Dicke des Widerlagers in der Mitte unterhalb der Schneidelektrode größer zu machen, als am Rand (an der Flanke bzw. Umfangsfläche).

Das Widerlager kann in Ruheposition von der Schneidelektrode weg oder auf diese hin gewölbt sein. Damit lassen sich verschiedene Federcharakteristika einstellen, um beim Einfedern, d.h. beim Schließen des Instruments, einen gewünschten Verlauf der Andruckkraft des Gewebes an die Schneidelektrode zu erzielen.

Bei einer vorteilhaften Ausführungsform schließt das Widerlager sowohl an seiner Oberseite als auch an seiner Unterseite bündig mit dem Rahmen ab. Dies erleichtert die Fertigung des Instruments, insbesondere die Erzeugung des Widerlagers in dem Rahmen.

Das Widerlager kann einen oder mehrere sich auf den Rahmen erstreckender Abschnitte aufweisen. Vorzugsweise ist ein solcher Abschnitt wenigstens an dem distalen Ende des Widerlagers bzw. der es enthaltenden Branche angeordnet. Solche Abschnitte können als Abstandshalter der Branchen dienen und verhindern, dass sich diese beim Schließen unmittelbar berühren. Abgesehen von diesen Abschnitten ist es aber vorteilhaft, wenn das Widerlager wenigstens ansonsten an der Oberseite und der Unterseite bündig mit dem Rahmen abschließt.

Weitere Einzelheiten vorteilhafter Ausführungsformen der Erfindung ergeben sich aus der Zeichnung, der zugehörigen Beschreibung sowie aus Ansprüchen. Es zeigen:
Figur 1 das erfindungsgemäße Instrument, in geschlossenem Zustand,
Figur 2 die obere und untere Branche des Instruments nach Figur 1, in geöffnetem Zustand, im Querschnitt,
Figur 3 das Instrument nach Figur 1 und 2 in geschlossenem Zustand, im Querschnitt ohne Gewebe,
Figur 4 das Instrument nach Figur 3 mit zwischen den Branchen gefasstem Gewebe, in Querschnittsdarstellung,
Figur 5 eine abgewandelte Ausführungsform des Instruments, in Querschnittsdarstellung in leicht geöffnetem Zustand,
Figur 6 eine weitere abgewandelte Ausführungsform des erfindungsgemäßen Instruments in Querschnittsdarstellung bei leicht geöffneten Branchen,
Figur 7 die untere Branche des Instruments nach Figur 6, in ausschnittsweiser Perspektivdarstellung und
Figur 8 eine Kunststoff-Spritzgussform zur Herstellung eines Widerlagers für das Instrument nach Figur 1 bis 5.

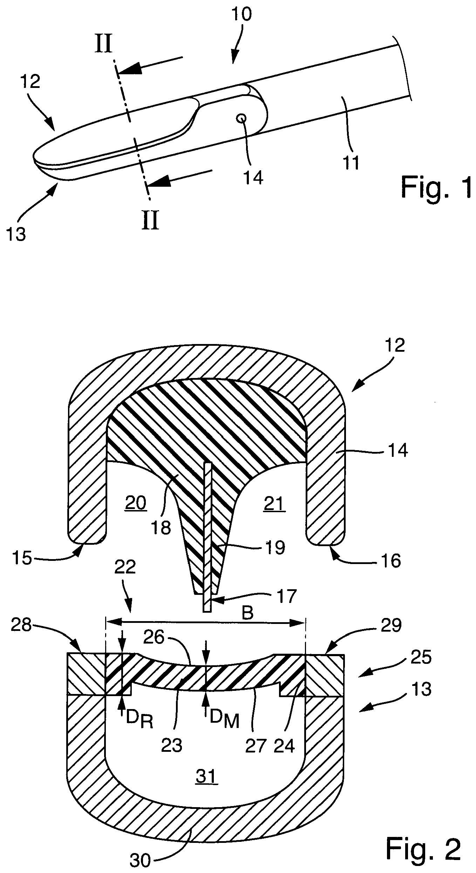

In Figur 1 ist ein Instrument veranschaulicht, das zur Gewebefusion und Dissektion eingerichtet ist. Bei dem Instrument 10 kann es sich um ein offenchirurgisch einzusetzendes Instrument, um ein laparoskopisch einzusetzendes Instrument oder auch um ein endoskopisch einzusetzendes Instrument handeln. Das Instrument 10 weist zwei beispielsweise an einem flexiblen Schaft 11 oder auch an einen steifen Schaft gehaltenen Branchen 12, 13 auf, von denen mindestens eine der Branchen, im vorliegenden Ausführungsbeispiel die obere Branche 12, schwenkbar gelagert ist, um auf die andere im vorliegenden Beispiel die untere Branche 13 hin und von dieser weg bewegt werden zu können. Die Schwenkbewegung um die Scharnierachse 14 wird von einem nicht weiter veranschaulichten sich durch den Schaft 11 erstreckenden Mechanismus gesteuert.

Alternativ kann das Instrument 10 auch zwei schwenkbar gelagerte Branchen aufweisen, die aufeinander zu und voneinander weg beweglich sind.

Figur 2 veranschaulicht das Instrument 10 nach Figur 1 im Querschnitt, geschnitten entlang der Linie II-II in Figur 1. Die obere Branche 12 kann zum Beispiel durch ein im Querschnitt U-förmiges außen metallisch blankes oder auch elektrisch isoliertes Metallteil 14 gebildet sein, dessen Schenkelstirnseiten 5 als Koagulationselektroden 15, 16 dienen. Die Koagulationselektroden 15, 16 können durchgehend streifenförmige Elektroden sein, sie können auch durch isolierende Abschnitte unterbrochen sein. Die obere Branche 12 kann anstelle eines Metallteils 14 auch ein anderes beispielsweise aus einem nicht leitendem Material, wie zum Beispiel Kunststoff bestehendes Teil aufweisen, in das die Koagulationselektroden 15, 16 beispielsweise in Gestalt von Blechteilen eingelassen sind.

Die obere Branche 12 ist mit einer Schneidelektrode 17 versehen, die zum Beispiel durch einen dünnen Blechstreifen gebildet ist. Vorzugsweise ist die Schneidelektrode 17 in einen Isolierkörper 18 etwa mittig zwischen den Koagulationselektroden 15, 16 gehalten. Die Schneidelektrode 17 ist vorzugsweise in einem Fortsatz 19 des Isolierkörpers 18 angeordnet, sodass die beiden Flachseiten der Schneidelektrode 19 isoliert sind. Zwischen dem Fortsatz 19 und den die Koagulationselektroden 15, 16 tragenden Schenkeln der oberen Branche 12 sind Freiräumen 20, 21 ausgebildet, die zur Gewebeaufnahme dienen.

Die untere Branche 13 enthält ein Widerlager 22, das dazu dient, biologisches Gewebe während eines Schneidvorgangs an der Schneidelektrode 17 zu halten. Das Widerlager 22 ist ein flexibler beispielsweise aus Silikon bestehender Körper, dessen in einem mittleren Bereich 23 insbesondere in Verlängerung der Schneidelektrode 17 zu messende Dicke D_{M} kleiner ist als die quer dazu zu messende Breite B. Das Widerlager 22 besteht aus Vollmaterial. Es weist vorzugsweise keinerlei Hohlräume auf.

Seitlich schließt das Widerlager 22 mit seiner Umfangsfläche 24 an einen Rahmen 25 an und ist dort zugfest stoffschlüssig durch Anspritzen, oder in einem nicht beanspruchten Beispiel durch Kleben, mit diesem verbunden. Die Umfangsfläche 24 ist vorzugsweise unstrukturiert, d.h. bis auf Bearbeitungsspuren wie Stanz- oder Bruchflächen, glatt. Sie kann jedoch auch strukturiert sein, um die stoffliche Verbindung zwischen dem Widerlager 22 und dem Rahmen 25 zum Beispiel durch Formschluss zu unterstützen.

Das Widerlager 22 weist an seinen an die Umfangsfläche 24 angrenzenden Rand eine Dicke D_{R} auf, die vorzugsweise mindestens so groß ist wie die Dicke D_{M} im mittleren Bereich 23. Die Dicke D_{R} entspricht dabei außerdem der in gleiche Richtung zu messende Dicke des Rahmens 25, sodass das Widerlager 22 sowohl an seiner Oberseite 26 als auch an seiner Unterseite 27 bündig mit dem Rahmen 25 abschließt.

Der Rahmen 25 besteht vorzugsweise aus Metall, sodass seine Oberseite die Koagulationselektroden 28, 29 bildet. Diese können an dem distalen Ende der jeweiligen Branche 12 bzw. 13 ineinander übergehen.

Die aus dem Widerlager 22 und dem Rahmen 25 bestehende Einheit kann in einem Spritzgussverfahren hergestellt werden, bei dem ein vorgefertigter Rahmen 25 in eine entsprechende Spritzgussform eingeführt wird, in die dann das Kunststoffmaterial des Widerlagers 22 eingeführt wird, um sich an der Umfangsfläche 24 innig mit dem Rand 25 zu verbinden. Die so gebildete Einheit ist mit dem übrigen tragenden Teil der unteren Branchen 13 verbunden, der im vorliegenden Ausführungsfall durch ein U-förmiges Metallteil 30 gebildet wird. Dieses kann an seiner Außenseite metallisch blank oder elektrisch isoliert zum Beispiel mit einem Kunststoffüberzug versehen sein. Auch der Rahmen 25 kann an seiner nach außen weisenden Seite metallisch blank, d.h. elektrisch leitende oder alternativ mit einer Isolierung versehen sein. Der Rahmen 25 kann mit dem Metallteil 30 elektrisch leitend verbunden oder auch gegen dieses isoliert sein. Zwischen dem Widerlager 22 und dem Metallteil 30 ist ein Innenraum 31 ausgebildet, der von der Umgebung getrennt über geeignete Öffnungen auch mit diese verbunden sein kann.

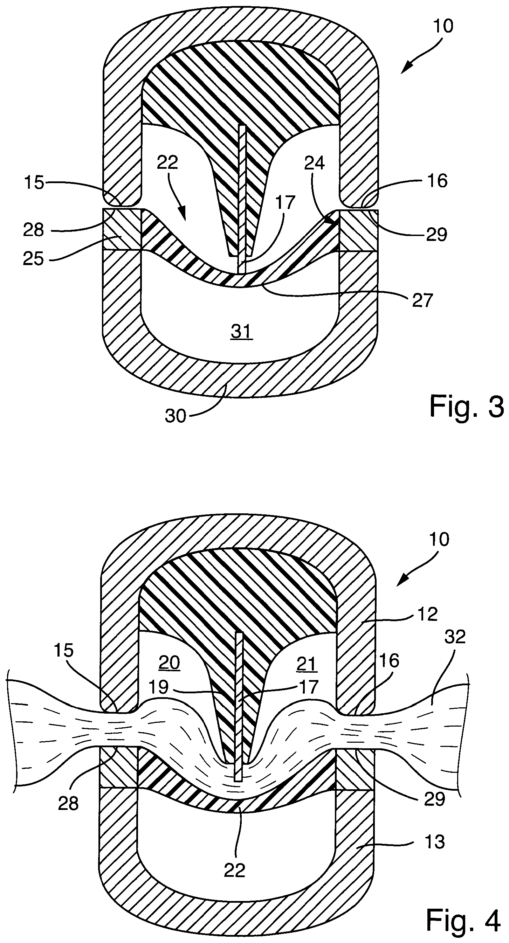

Figur 3 veranschaulicht das Instrument 10 in geschlossenem Zustand. Die Elektroden 15, 28 berühren einander oder sind über nicht weiter dargestellte Abstandshalter in geringem Abstand zueinander gehalten. Gleiches gilt für die Koagulationselektroden 16, 29. Die Schneidelektrode 17 steht auf dem Widerlager 22 und drückt dieses in den Freiraum 31 hinein, ohne dass die Unterseite 27 des Widerlagers 22 das Metallteil 30 oder irgendein anderes in dem Freiraum 31 befindliches Teil berührt. Weil das Widerlager 22 im Querschnitt schlank ist, d.h. weil seine Dicke D_{M} wesentliche geringer ist als seine Breite B, wird das Widerlager 22 bei der in Figur 3 veranschaulichten Deformation im Wesentlichen auf Zug beansprucht. Es wird gedehnt. Ebenso wird die Verbindung zwischen seiner Umfangsfläche 24 und dem Rahmen 25 im Wesentlichen auf Zug beansprucht.

Das Instrument 10 arbeitet wie folgt:
Wie Figur 4 veranschaulicht, wird zur Behandlung von biologischem Gewebe 32 dieses zunächst zwischen den Branchen 12, 13 gefasst. Das biologische Gewebe 32 kann Organgewebe, ein Blutgefäß oder auch anderweitiges Gewebe sein. Die aufeinander zu bewegten Branchen 12, 13 fassen das Gewebe 32. Gleichzeitig oder in zeitlicher Reihenfolge werden die Koagulationselektroden 15, 28, 16, 29 mit Koagulationsspannung und die Schneidelektrode 17 mit Schneidspannung beaufschlagt. Das Widerlager 22 ist, wie Figur 2 zeigt, beim Schließen des Instruments 10 der Schneidelektrode 17 und dem Gewebe 32 ausgewichen und dadurch gespannt worden. Das Widerlager drängt das Gewebe 32 dabei aber an die Schneidelektrode 17. Das biologische Gewebe 32 kann zu beiden Seiten des Fortsatzes 19 in die Freiräume 20,21 eindringen. Die Koagulationselektroden 15, 28 sowie 16, 29 koagulieren das zwischen ihnen gefasste Gewebe und falls es sich um ein Gefäß handelt, fusionieren sie es, d.h. schließen an diesen Stellen. Das von der Schneidelektrode 17 mit einer entsprechend höheren Schneidspannung beaufschlagt Gewebe schrumpft und trennt sich, wobei das Widerlager 22 das schrumpfende Gewebe sich an der Schneidelektrode 17 hält.

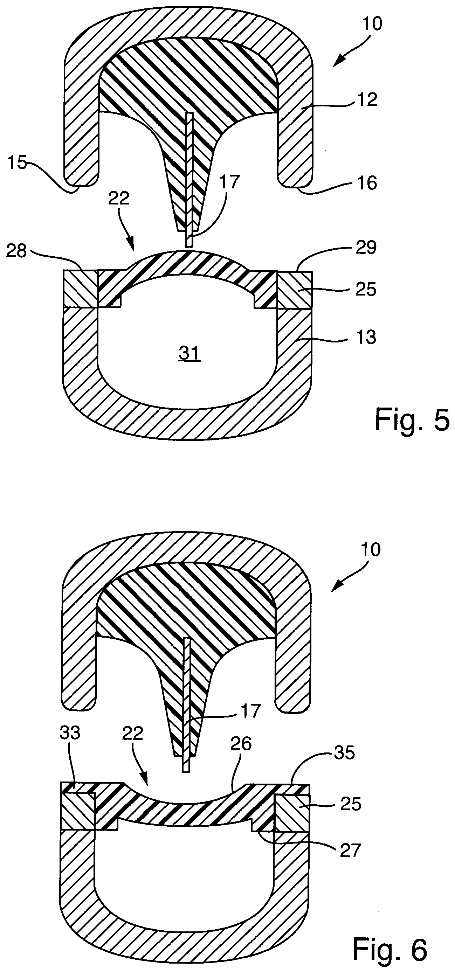

An der insoweit beschriebenen Erfindung sind Abwandlungen möglich. Beispielsweise kann, wie Figur 5 veranschaulicht, das Widerlager 22 zu der Schneidelektrode 17 hin konvex gewölbt sein. Dies führt dazu, dass beim Schließen des Instruments 10 zunächst die Wölbung des Widerlagers 22 überwunden werden muss, wodurch ein gewisser Schnappeffekt auftreten kann. Alternativ kann das Widerlager 22 auch vollständig eben sein. Ansonsten gilt die Beschreibung der Figuren 1 bis 4 für die Ausführungsform des Instruments 10 nach Figur 5 entsprechend.

Das Widerlager 22 kann auch eben oder wie bei den Ausführungsformen nach Figur 1 bis 4 sowie konkav, d.h. von der Schneidelektrode 17 weg gewölbt ausgebildet sein. Mit dem Verlauf der Dicke von der Mitte 23 zum Rand des Widerlagers 22 sowie mit der Wölbung lassen sich gewünschte Federkennlinien, d.h. Weg-Kraft-Verläufe einstellen, die beim Schließen des Instruments 10 zu verzeichnen sind.

Bei allen vorbeschriebenen Ausführungsformen kann das Widerlager 22 Fortsätze 33, 34, 35 aufweisen, die den Rahmen 25 übergreifen und somit eine direkte Berührung der Koagulationselektroden 15, 16 mit den Koagulationselektroden 28, 29 verhindern. Im Übrigen schließt das Widerlager 22 jedoch auch bei dieser Ausführungsform vorzugsweise bündig sowohl an seiner Oberseite 26 als auch an seiner Unterseite 27 bündig mit dem Rahmen 25 ab.

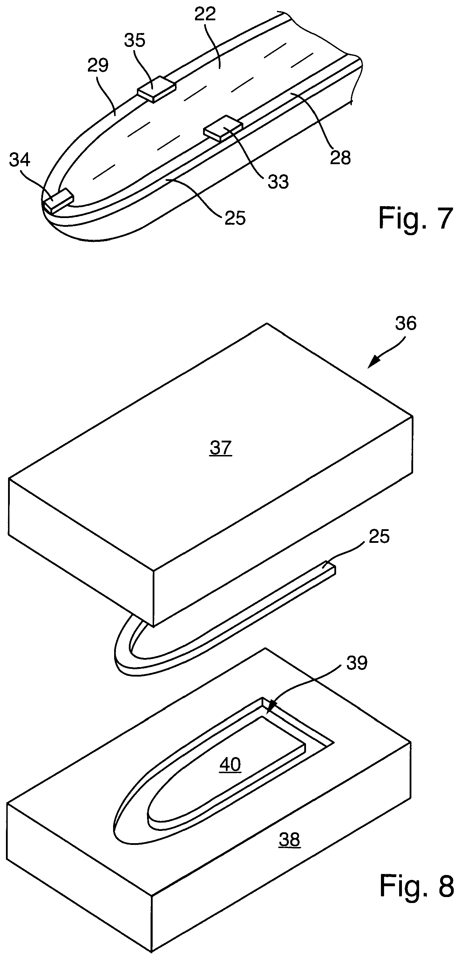

Die Herstellung des Widerlagers 22 soll anhand von Figur 8 veranschaulicht werden. Es wird dazu eine Kunststoff-Spritzgussform 36 bereitgestellt, zwischen deren oberer Formhälfte 37 und deren unterer Formhälfte 38 eine Gravur 39 ausgebildet ist, die die Form des zu erzeugenden Widerlagers festlegt. In Figur 8 ist in der Gravur 39 ein mittlerer erhabener Vorsprung 40 veranschaulicht, der die Dicke des zu erzeugenden Widerlagers 22 im mittleren Beriech reduziert.

Zur Herstellung des Widerlagers 22 wird bei geöffneter Kunststoff-Spritzgussform 36 der Rahmen 25 in die Gravur 39 eingelegt, wonach die Spritzgussform 36 geschlossen wird. Danach wird flüssiger Kunststoff in die Gravur 39 eingefüllt, sodass dieser den vorhandenen Raum restlos füllt und sich an die Innenseite des Rahmens 25 anlegt und adhäsiv mit dieser verbindet. Nach dem Aushärten des Kunststoffs kann die Kunststoff-Spritzgussform 36 geöffnet und das Widerlager 22 entnommen werden.

Bei einem erfindungsgemäßen Instrument 10, das vorzugsweise zur Gewebefusion und Dissektion einsetzbar ist, ist einer zwischen zwei Elektrodenpaaren angeordneten Schneidelektrode 17 ein Widerlager 22 zugeordnet, das als dünne Membran ausgebildet ist. Die dünne Membran ist in einem Rahmen 25 gespannt, an dem sie mit ihrer Umfangsfläche stoffschlüssig gehalten ist. Die Umfangsfläche steht dabei in einem Winkel, vorzugsweise einem rechten Winkel, zu dem von der Rahmenoberseite gebildeten Koagulationselektroden 28, 29. Die von der Membran ausgeübte Andruckkraft, mit der das biologische Gewebe 32 an die Schneidelektrode 17 angedrückt wird, wird überwiegend von der in der Membran 22 vorhandenen Zugspannung erzeugt.

### Bezugszeichen:

- 10: Instrument
- 11: Schaft
- 12: obere Branche
- 13: untere Branche
- 14: Metallteil
- 15, 16: Koagulationselektroden der oberen Branche 12
- 17: Schneidelektrode
- 18: Isolierkörper
- 19: Fortsatz des Isolierkörpers
- 20, 21: Freiräume
- 22: Widerlager
- 23: mittlerer Bereich des Widerlagers 22
- 24: Umfangsfläche des Widerlagers
- 25: Rahmen
- 26: Oberseite des Widerlagers 22
- 27: Unterseite des Widerlagers
- 28, 29: Koagulationselektroden der unteren Branche 13
- 30: Metallteil
- 31: Freiraum
- 32: biologisches Gewebe
- 33 - 35: Fortsätze
- 36: Kunststoff-Spritzgussform
- 37: obere Formhälfte
- 38: untere Formhälfte
- 39: Gravur
- 40: Vorsprung

## Patentansprüche

1. Instrument (10), insbesondere zur Dissektion oder zur kombinierten Gewebefusion oder -koagulation und Gewebedissektion,
mit zwei Branchen (12, 13), von denen wenigstens eine auf die andere hin und von dieser weg beweglich gelagert ist,
mit einer Schneidelektrode (17), die an einer der Branchen (12) angeordnet ist,
mit einem der Schneidelektrode (17) zugeordneten elastischen Widerlager (22), das an der anderen der Branchen (13) angeordnet und in einem Rahmen (25) gehalten ist, an dem es mit seiner Umfangsfläche (24) anliegend angeordnet ist,
**dadurch gekennzeichnet,**
**dass** das Widerlager (22) an seiner Umfangsfläche (24) an den Rahmen (25) anschließt und dort stoffschlüssig durch Anspritzen mit dem Rahmen (25) zugfest verbunden ist.

2. Instrument nach Anspruch 1, **dadurch gekennzeichnet, dass** das Widerlager (22) in dem Rahmen (25) unterstützungsfrei gehalten ist.

3. Instrument nach einem der vorstehenden Ansprüche,
**dadurch gekennzeichnet, dass** das Widerlager (22) eine Kunststoffmembrane ist, deren in Bewegungsrichtung der wenigstens einen beweglichen Branche (12) zu messende Dicke (D_{M}) in einem mittleren Bereich (23), auf den die Schneidelektrode (17) in Betrieb aufsetzt, höchstens so groß ist, wie an der Umfangsfläche (24).

4. Instrument nach einem der vorstehenden Ansprüche, **dadurch gekennzeichnet, dass** das Widerlager (22) eine Kunststoffmembrane ist, deren in Bewegungsrichtung der wenigstens einen beweglichen Branche (12) zu messende Dicke (D_{M}) in einem mittleren Bereich (23), auf den die Schneidelektrode (17) in Betrieb aufsetzt, eine geringere Dicke aufweist, als an allen anderen Stellen.

5. Instrument nach einem der vorstehenden Ansprüche, **dadurch gekennzeichnet, dass** das Widerlager (22) eine Kunststoffmembrane ist, deren in Bewegungsrichtung der wenigstens einen beweglichen Branche (12) zu messende Dicke (D_{M}) in einem mittleren Bereich (23), auf den die Schneidelektrode (17) in Betrieb aufsetzt, eine größere Dicke aufweist, als an allen anderen Stellen.

6. Instrument nach einem der vorstehenden Ansprüche, **dadurch gekennzeichnet, dass** das Widerlager (22) im Ruhezustand an seiner der Schneidelektrode (17) zugewandten Seite (26) konkav oder konvex gewölbt ausgebildet ist.

7. Instrument nach einem der vorstehenden Ansprüche, **dadurch gekennzeichnet, dass** das Widerlager (22) im die von dem Rahmen (25) umgrenzte Öffnung ganz verschließend ausgebildet und angeordnet ist.

8. Instrument nach einem der vorstehenden Ansprüche,
**dadurch gekennzeichnet, dass** das Widerlager (22) sowohl an seiner Oberseite (26) als auch an seiner Unterseite (27) bündig mit dem Rahmen (25) abschließend ausgebildet ist.

9. Instrument nach einem der vorstehenden Ansprüche,
**dadurch gekennzeichnet, dass** das Widerlager (22) wenigstens einen sich auf den Rahmen (25) erstreckenden Abschnitt (33, 34, 35) aufweist.

10. Instrument nach Anspruch 9, **dadurch gekennzeichnet, dass** der Abschnitt (34) an dem distalen Ende der das Widerlager (22) enthaltenden Branche (13) angeordnet ist.

11. Verfahren zur Herstellung eines Widerlagers für ein Instrument nach Anspruch 1, mit folgenden Schritten:
Bereitstellung einer Kunststoff-Spritzgussform (36) mit einer Gravur (39) zur Aufnahme des Rahmens (25),
Anordnen des Rahmens (25) in der Gravur (39),
Einleiten von Kunststoff in die Gravur (39), so dass dieser sich an den Rahmen (25) anlegt und adhäsiv verbindet,
Erstarren lassen des Kunststoffs so dass dieser zusammen mit dem Rahmen (25) das Widerlager (22) bildet,
und
Öffnen der Spritzgussform (36) und Entnehmen des Widerlagers (22).

## Claims

1. Instrument (10), particularly for cutting or for combined tissue fusion or coagulation and tissue cutting,
having two jaws (12, 13) at least one of which is movably supported toward and away from the other,
having a cutting electrode (17) that is arranged on one of the jaws (12),
having an elastic counter support (22) assigned to the cutting electrode (17) that is arranged on the other jaw (13) and is held in a frame (25) on which it is arranged being in abutment with its circumferential surface (24),
**characterized in that**
the counter support (22) adjoins the frame (25) at its circumferential surface (24) and is there connected with the frame (25) in a tensile-resistant manner by a substance bond formed by overmolding.

2. Instrument according to claim 1, **characterized in that** the counter support (22) is held without support in the frame (25).

3. Instrument according to any of the preceding claims, **characterized in that** the counter support (22) is a plastic membrane, wherein the thickness (D_{M}) to be measured in movement direction of the at least one movable jaw (12) in a central section (23) of the plastic membrane, on which the cutting electrode (17) is placed during operation, is at most as large as at the circumferential surface (24).

4. Instrument according to any of the preceding claims, **characterized in that** the counter support (22) is a plastic membrane, wherein the thickness (D_{M}) to be measured in movement direction of the at least one movable jaw (12) in a central section (23) of the plastic membrane, on which the cutting electrode (17) is placed during operation, has a smaller thickness than at all other locations.

5. Instrument according to any of the preceding claims, **characterized in that** the counter support (22) is a plastic membrane, wherein the thickness (D_{M}) to be measured in movement direction of the at least one movable jaw (12) in a central section (23) of the plastic membrane, on which the cutting electrode (17) is placed during operation, has a larger thickness than at all other locations.

6. Instrument according to any of the preceding claims, **characterized in that** the counter support (22) is formed in a concave or convex domed manner in its rest condition on its side facing the cutting electrode (17).

7. Instrument according to any of the preceding claims, **characterized in that** the counter support (22) is configured and arranged in the opening limited by the frame (25) in a manner to entirely close the opening.

8. Instrument according to any of the preceding claims, **characterized in that** the counter support (22) is configured to end flush with the frame (25) at its top side (26) as well as its bottom side (27).

9. Instrument according to any of the preceding claims, **characterized in that** the counter support (22) comprises a section (33, 34, 35) extending on the frame (25).

10. Instrument according to claim 9, **characterized in that** the section (34) is arranged at the distal end of the jaw (13) comprising the counter support (22).

11. Method for manufacturing a counter support for an instrument according to claim 1, comprising the following steps:
providing a plastic injection molding die (36) having an engraving (39) for locating the frame (25) therein,
arranging the frame (25) in the engraving (39),
introducing plastic in the engraving (39) such that it contacts and adhesively connects with frame (25),
solidifying the plastic such that it forms the counter support (22) together with the frame (25), and
opening the injection molding die (36) and removing the counter support (22).

## Revendications

1. Instrument (10), destiné en particulier à la dissection ou à la fusion ou la coagulation tissulaire combinée et la dissection tissulaire,
comprenant deux mors (12, 13) dont au moins un est monté de façon mobile, de manière à pouvoir être rapproché et éloigné de l'autre,
comprenant une électrode de coupe (17) qui est disposée sur l'un des mors (12),
comprenant une butée (22) élastique qui est associée à l'électrode de coupe (17) et est disposée sur l'autre mors (13) et est tenue dans un cadre (25) sur lequel elle est disposée en étant appliquée avec sa surface périphérique (24),
**caractérisé en ce que**
la butée (22) se raccorde au cadre (25) avec sa surface périphérique (24) et est reliée à cet endroit au cadre (25) par matière, par injection, de manière à résister à la traction.

2. Instrument selon la revendication 1, **caractérisé en ce que** la butée (22) est tenue dans le cadre (25) sans soutien.

3. Instrument selon une des revendications précédentes, **caractérisé en ce que** la butée (22) est une membrane en matière plastique dont l'épaisseur (D_{M}) devant être mesurée dans la direction de déplacement du mors (12) mobile, au nombre d'au moins un, est au maximum aussi grande qu'au niveau de la surface périphérique (24), dans une zone centrale (23) sur laquelle l'électrode de coupe (17) se pose lors du fonctionnement.

4. Instrument selon une des revendications précédentes, **caractérisé en ce que** la butée (22) est une membrane en matière plastique dont l'épaisseur (D_{M}) devant être mesurée dans la direction de déplacement du mors (12) mobile, au nombre d'au moins un, présente une épaisseur qui est inférieure à celle de tous les autres emplacements, dans une zone centrale (23) sur laquelle l'électrode de coupe (17) se pose lors du fonctionnement.

5. Instrument selon une des revendications précédentes, **caractérisé en ce que** la butée (22) est une membrane en matière plastique dont l'épaisseur (D_{M}) devant être mesurée dans la direction de déplacement du mors (12) mobile, au nombre d'au moins un, présente une épaisseur qui est supérieure à celle de tous les autres emplacements, dans une zone centrale (23) sur laquelle l'électrode de coupe (17) se pose lors du fonctionnement.

6. Instrument selon une des revendications précédentes, **caractérisé en ce que**, à l'état de repos, la butée (22) présente une courbure concave ou convexe sur sa face (26) tournée vers l'électrode de coupe (17).

7. Instrument selon une des revendications précédentes, **caractérisé en ce que** la butée (22) est réalisée et disposée de manière à fermer complètement l'ouverture délimitée par le cadre (25).

8. Instrument selon une des revendications précédentes, **caractérisé en ce que** la butée (22) est réalisée, aussi bien sur sa face supérieure (26) que sur sa face inférieure (27), de manière à affleurer avec le cadre (25).

9. Instrument selon une des revendications précédentes, **caractérisé en ce que** la butée (22) comporte au moins une partie (33, 34, 35) qui s'étend sur le cadre (25).

10. Instrument selon la revendication 9, **caractérisé en ce que** la partie (34) est disposée sur l'extrémité distale du mors (13) contenant la butée (22).

11. Procédé de fabrication d'une butée destinée à un instrument selon la revendication 1, comprenant les étapes suivantes :
mise à disposition d'un moule d'injection de matière plastique (36) avec une gravure (39) destinée à recevoir le cadre (25),
mise en place du cadre (25) dans la gravure (39),
introduction de matière plastique dans la gravure (39), de manière à ce qu'elle s'applique contre le cadre (25) et le relie par adhésion,
rigidification de la matière plastique, de manière à ce que celle-ci forme la butée (22), conjointement avec le cadre (25),
et
ouverture du moule à injection (26) et retrait de la butée (22).
